# EUROPEAN PATENT SPECIFICATION

(11) **EP 2 509 945 B1**
(45) Date of publication and mention of the grant of the patent: **05.03.2014**
(21) Application number: 10787772.2
(22) Date of filing: 08.12.2010
(51) Int. Cl.: C07D 207/48, C07D 211/96, A61K 31/451, A61P 17/00

(54) **4-(AZACYCLOALKYL)-BENZENE-1,3-DIOL DERIVATIVES AS TYROSINASE INHIBITORS AND THEIR SYNTHESIS AND USE THEREOF**
4- (AZACYCLOALKYL) -BENZOL-1, 3 -DIOL-DERIVATE ALS TYROSINASEHEMMER, IHRE SYNTHESE UND VERWENDUNG
DÉRIVÉS DE 4-(AZACYCLOALKYL)-BENZÈNE-1,3-DIOL EN TANT QU'INHIBITEURS DE TYROSINASE ET LEUR SYNTHÈSE ET UTILISATION DE CEUX-CI

(30) Priority: 10.12.2009 US 282064 P; 10.12.2009 FR 0958829
(43) Date of publication of application: 17.10.2012
(73) Proprietor: Galderma Research & Development, 06410 Biot (FR)
(72) Inventor: BOITEAU, Jean-Guy, F-06560 Valbonne (FR); LUZY, Anne-Pascale, F-06560 Valbonne (FR)
(74) Representative: Tezier Herman, Béatrice
(86) International application number: PCT/EP2010/069195
(87) International publication number: WO 2011/070080

(56) References cited:
- WO-A2-02/20474

## Description

The invention relates to novel 4-(azacycloalkyl)-benzene-1,3-diol compounds as industrial and consumer products. It also relates to a method for their preparation and to their use, as tyrosinase inhibitors, in pharmaceutical or cosmetic compositions for the treatment or prevention of pigmentary disorders.

Pigmentation of the skin, especially human skin, results from the synthesis of melanin by dendritic cells known as melanocytes. Melanocytes contain organelles known as melanosomes which transfer melanin into the upper keratinocyte layers which are then transported to the surface of the skin by differentiation of the epidermis (Gilchrest B A, Park H Y, Eller M S, Yaar M, Mechanisms of ultraviolet light-induced pigmentation. Photochem Photobiol 1996; 63: 1-10; Hearing V J, Tsukamoto K, Enzymatic control of pigmentation in mammals. FASEB J 1991; 5: 2902-2909).

Tyrosinase is a key enzyme among melanogenesis enzymes which catalyses the first two steps of melanin synthesis. Homozygotic mutations of tyrosinase cause oculocutaneous albinism type I, characterized by a complete absence of melanin synthesis (Toyofuku K, Wada I, Spritz R A, Hearing V J, The molecular basis of oculocutaneous albinism type 1 (OCA1) : sorting failure and degradation of mutant tyrosinases results in a lack of pigmentation. Biochem J 2001; 355: 259-269).

In order to treat pigmentation disorders resulting from an increase in melanin production for which no treatment exists that satisfies all of the expectations of patients and dermatologists alike, the development of novel therapeutic approaches has proved to be important.

The majority of known skin lightening compounds are phenols or hydroquinone derivatives. Those compounds inhibit tyrosinase, but the majority of them are cytotoxic as regards melanocytes. This toxic effect risks causing permanent depigmentation of the skin. Producing compounds that could inhibit melanogenesis while remaining low in cytotoxicity or devoid of toxicity as regards melanocytes would be of particular interest.

Among the compounds which have been described in the literature, patent application WO99/15148 discloses the use of 4-cycloalkylresorcinols as a depigmentation agent.

Patent FR 2 704 428 discloses the use of 4-haloresorcinols as depigmentation agents.

Patent applications WO2006/097224 and WO2006/097223 disclose the use of 4-cycloalkylmethylresorcinols as depigmentation agents.

Patent application WO2005/085169 discloses the use of alkyl 3-(2,4-dihydroxyphenyl)propionates as depigmentation agents.

Patent application WO2004/017936 discloses the use of 3-(2,4-dihydroxyphenyl)acrylamide as a depigmentation agent.

Patent application WO2004/052330 discloses the use of 4-[1,3]dithian-2-ylresorcinols as depigmentation agents.

WO 02/20474 discloses the use of 4-cycloalkylresorcinols as skin lightening agents.

More particularly, patent EP 0 341 664 discloses the use of 4-alkylresorcinols as depigmentation agents, among them 4-n-butyl resorcinol, also known as Rucinol, which forms part of the composition of a depigmentation cream sold under the trade name Iklen®.

Surprisingly and unexpectedly, the Applicant has now discovered that novel compounds with a 4-(azacycloalkyl)benzene-1,3-diol structure have very good tyrosinase enzyme inhibiting activity and very low cytotoxicity. Furthermore, these compounds have a tyrosinase enzyme inhibiting activity which is greater than that of Rucinol while being less cytotoxic as regards melanocytes than Rucinol.

These compounds may have applications in human medicine, especially in dermatology and in the cosmetics field.

Thus, the present invention concerns compounds with the following general formula (I): 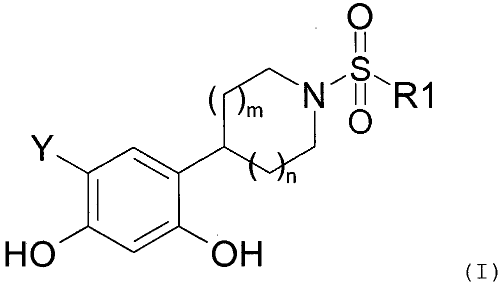 wherein
R1 represents:
- a C1-C8 alkyl radical;
- a C3-C8 cycloalkyl radical;
- a C4-C10 methylcycloalkyl radical;
- an aryl radical;
- a substituted aryl radical;
- a heteroaryl radical;
- a substituted heteroaryl radical;
- an aralkyl radical; or
- a C1-C5 alkoxy radical;
Y represents hydrogen or fluorine; and m and n may independently take the values 0, 1 or 2; as well as salts of the compounds with formula (I), and their enantiomeric forms.

Preferred salts of the compounds with general formula (I) with a pharmaceutically acceptable base which may be cited are salts with an organic base or with an inorganic base.

Examples of suitable inorganic bases are potassium hydroxide, sodium hydroxide or calcium hydroxide.

Examples of suitable organic bases are morpholine, piperazine and lysine.

The compounds with general formula (I) may also exist in hydrated or solvated forms.

Examples of suitable solvents for forming solvates are alcohols such as ethanol or isopropanol.

According to the present invention, the term "C1-C8 alkyl" denotes a saturated linear or branched hydrocarbon chain containing 1 to 8 carbon atoms.

According to the present invention, the term "C3-C8 cycloalkyl" denotes a saturated cyclic hydrocarbon chain containing 3 to 8 carbon atoms.

According to the present invention, the term "C4-C10 methylcycloalkyl" denotes methyl substituted with a saturated, cyclic or bicyclic hydrocarbon chain containing 3 to 9 carbon atoms, which may be substituted with an oxygen atom.

According to the present invention, the term "aryl" means a phenyl or a naphthyl group.

According to the present invention, the term "substituted aryl" means a phenyl or a naphthyl group substituted with one or more groups of atoms selected from a C1-C8 alkyl, a C1-C5 alkoxy, a halogen and a trifluoromethyl group.

According to the present invention, the term "heteroaryl" means a pyridine or a quinoline group.

According to the present invention, the term "substituted heteroaryl" means a pyridine or a quinoline group substituted with one or more groups of atoms selected from a C1-C8 alkyl, a C1-C5 alkoxy, a halogen and a trifluoromethyl group.

Preferred possible substitutions on a phenyl, a naphthyl, a pyridine or a quinoline group are those where said radicals are substituted with methyl (C1 alkyl).

According to the present invention, the term "aralkyl" denotes a C1-C8 alkyl radical as defined above and substituted with a substituted or unsubstituted aryl radical.

According to the present invention, the term "C1-C5 alkoxy" denotes an oxygen atom substituted with a linear or branched saturated hydrocarbon chain containing 1 to 5 carbon atoms.

According to the present invention, the term "halogen" denotes a chlorine, fluorine, bromine or iodine atom.

According to the present invention, particularly preferred compounds with general formula (I) are those wherein R1 represents:
- a substituted aryl radical;
- an aralkyl radical;
- a C3-C8 cycloalkyl radical; or
- a C4-C10 methylcycloalkyl radical;
Y represents hydrogen or fluorine; and
m and n may independently take the values 0, 1 or 2;
as well as salts of these compounds with general formula (I) and their enantiomeric forms.

According to the present invention, more particularly preferred compounds with general formula (I) are those wherein R1 represents:
- a substituted aryl radical;
- an aralkyl radical;
- a C3-C8 cycloalkyl radical; or
- a C4-C10 methylcycloalkyl radical;
- Y represents hydrogen or fluorine; and
- m = 1 and n = 1;
as well as salts of these compounds with general formula (I) and their enantiomeric forms.

The following examples of compounds with formula (I) that fall within the scope of the present invention may in particular be cited:
1: 4-(1-phenylmethanesulphonylpiperidin-4-yl)-benzene-1,3-diol;
2: 4-[1-(toluene-4-sulphonyl)piperidin-4-yl]-benzene-1,3-diol;
3: 4-[1-(butane-1-sulphonyl)piperidin-4-yl]benzene-1,3-diol;
4: 4-(1-cyclohexylmethanesulphonylpiperidin-4-yl)-benzene-1,3-diol;
5: 4-[1-(2-phenylethanesulphonyl)piperidin-4-yl]-benzene-1,3-diol;
6: 1-[4-(2,4-dihydroxyphenyl)piperidine-1-sulphonylmethyl]-7,7-dimethyl-bicyclo[2.2.1]heptan-2-one;
7: 4-fluoro-6-[1-(toluene-4-sulphonyl)piperidin-4-yl]benzene-1,3-diol;
8: 4-fluoro-6-(1-phenylmethanesulphonylpiperidin-4-yl)benzene-1,3-diol;
9: 4-fluoro-6-[1-(2-phenylethanesulphonyl)piperidin-4-yl]benzene-1,3-diol;
10: 4-(1-cyclohexylmethanesulphonylpiperidin-4-yl)-6-fluorobenzene-1,3-diol;
11: 4-[1-(butane-1-sulphonyl)piperidin-4-yl]-6-fluorobenzene-1,3-diol;
12: 4-[1-(toluene-4-sulphonyl)pyrrolidin-3-yl]-benzene-1,3-diol;
13: 4-(1-phenylmethanesulphonylpyrrolidin-3-yl)-benzene-1,3-diol;
14: 4-[1-(2-phenylethanesulphonyl)pyrrolidin-3-yl]-benzene-1,3-diol;
15: 4-fluoro-6-[1-(2-phenylethanesulphonyl)-pyrrolidin-3-yl]benzene-1,3-diol;
16: 4-(1-cyclohexylmethanesulphonylpyrrolidin-3-yl)-benzene-1,3-diol;
17: 4-[1-(butane-1-sulphonyl)pyrrolidin-3-yl]-benzene-1,3-diol.

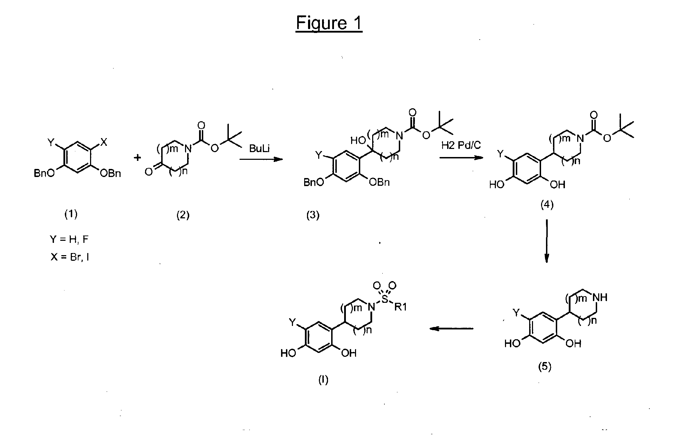

The compounds with general formula (I) are prepared using the general reaction scheme shown in Figure 1.

The compounds 2,4-bisbenzyloxybromobenzene (X=Br; Y=H) or 1,5-bisbenzyloxy-2-fluoro-4-iodobenzene (X=I; Y=F) (1), either commercially available or prepared using conventional synthesis methods (W D Langley, Org Synth I, 122 (1932) or, in the case of the fluorinated compounds: Mottram L F; Boonyarattanakalin S; Kovel R E; Peterson B R Organic Letters 2006, 8(4), 581-584), react in the presence of butyl lithium, for example with azacycloalkanones (2), commercially available or prepared using conventional synthesis methods (W D Langley, Org Synth I, 122 (1932)) to provide the corresponding benzyl alcohols with general formula (3) in which Y=H or F (Annoura H; Nakanishi K; Uesugi M; Fukunaga A; Imajo S; Miyajima A; Tamura-Horikawa Y; Tamura S; Bioorg Med Chem 2002, 10 (2), 371-383).

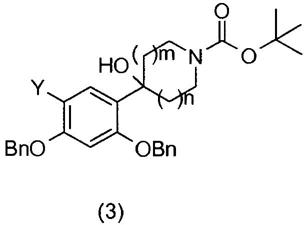

The compounds with general formula (4) are obtained by hydrogenation of benzyl alcohols with general formula (3) in the presence of hydrogen and a palladium-based catalyst such as palladium on charcoal, for example in a solvent such as methanol (Merschaert A; Delhaye L; Ketesmont J-P; Brione W; Delbeke P; Mancuso V; Napora F; Diker K; Giraud D; Vanmarsenille M; Tetrahedron Lett 2003, 44 (24), 4531-4534).

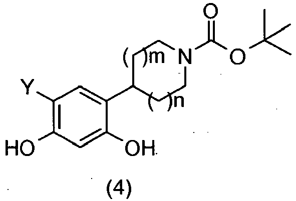

The compounds with general formula (4) are transformed into amines with general formula (5) by the action of trifluoroacetic acid in a solvent such as dichloromethane, for example (Kasyan A; Wagner C; Maier M E; Tetrahedron 1998, 54 (28), 8047-8054) or by the action of hydrogen chloride in solution in ethyl acetate, for example.

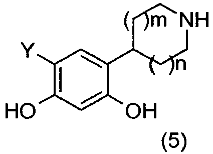

The compounds with general formula (5) are then transformed into compounds with general formula (I) by reaction with a derivative comprising a chlorosulphonyl function, for example in a solvent such as DMF in the presence of a base such as diisopropylamine, for example.

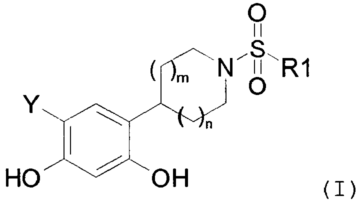

The invention thus envisages at least a compound of formula (I) for use as a drug.

The invention also envisages at least one compound with general formula (I) as hereinbefore defined for use, as a drug, in which said compound has a tyrosinase inhibiting activity.

The invention also envisages the use of at least one compound with general formula (I) as hereinbefore defined for the preparation of a pharmaceutical or cosmetic composition in which said compound has a tyrosinase inhibiting activity.

Advantageously, the compounds of the present invention have an IC50 value (dose inhibiting 50% of enzymatic activity) towards tyrosinase of 10 µM or less, more particularly 1 µM or less.

The invention also concerns a compound with general formula (I) for its use in the treatment and/or prevention of pigmentary disorders.

The compounds with general formula (I) of the invention are particularly suitable for use connected with the treatment and/or prevention of pigmentary disorders such as melasma, chloasma, lentigines, senile lentigo, irregular hyperpigmentations linked to photo-ageing, freckles, post-inflammatory hyperpigmentations due to abrasion, a burn, a scar, dermatosis, a contact allergy; naevi, hyperpigmentations with genetic determinism, hyperpigmentations of metabolic or drug origin, melanomas or any other hyperpigmentary lesion.

The present invention also pertains to a pharmaceutical composition in particular for the treatment of the disorders cited above, which is characterized in that it comprises, in a pharmaceutically acceptable support which is compatible with the mode of administration used therewith, a compound with general formula (I) in one of its isomeric or enantiomeric forms, or one of its salts with a pharmaceutically acceptable base.

The term "pharmaceutically acceptable support" means a medium which is compatible with the skin, mucosae, hair and nails.

The composition of the invention may be administered topically. Preferably, the pharmaceutical composition is packaged in a form which is suitable for topical application.

The term "topical application" means that the pharmaceutical composition of the invention is more particularly intended for the treatment of the skin and the mucosae and may be in the form of a liquid, paste or solid, more particularly in the form of ointments, creams, solutions or gels.

The compositions used for topical application have a concentration of the compound of the invention which is generally in the range 0.001% to 10% by weight, preferably in the range 0.01% to 5% by weight with respect to the total composition weight.

The compounds with general formula (I) of the invention also have an application in the cosmetic field, in particular in protection against the deleterious effect of the sun, in order to prevent and/or combat photo-induced or chronological ageing of the skin, and also to lighten skins with a dark phototype.

The invention thus also pertains to a composition comprising at least one of the compounds with formula (I), in a cosmetically acceptable support. The term "cosmetically acceptable medium" means a medium which is compatible with the skin, mucosae, hair and nails.

The invention also pertains to the cosmetic use of a composition comprising at least one compound with general formula (I), to prevent and/or treat signs of ageing and/or the skin.

The invention also pertains to the cosmetic use of a composition comprising at least one compound with general formula (I) for body or hair care.

The cosmetic composition of the invention containing a compound with general formula (I) or one of its isomeric or enantiomeric forms or one of its salts with a cosmetically acceptable base in a cosmetically acceptable support may be in the form of a cream, a milk, a gel, suspensions of microspheres or nanospheres or lipid or polymeric vesicles, impregnated pads, solutions, sprays, foams, sticks, soaps, wash bases or shampoos.

The concentration of the compound with general formula (I) in the cosmetic composition is preferably in the range 0.001% to 10% by weight with respect to the total composition weight.

The pharmaceutical and cosmetic compositions as described above may also contain inert additives or even pharmacological active ingredients in the pharmaceutical compositions, or combinations of such additives, and in particular:
- wetting agents;
- taste improving agents;
- preservatives, such as parahydroxybenzoic acid esters;
- stabilizing agents;
- moisture regulating agents;
- pH regulating agents;
- osmotic pressure modifying agents;
- emulsifying agents;
- UV-A and UV-B screens;
- antioxidants such as α-tocopherol, butylhydroxyanisole or butylhydroxytoluene, superoxide dismutase, or ubiquinol; sodium metabisulphite;
- emollients;
- moisturizers such as glycerol, PEG 400, thiamorpholinone and its derivatives, or urea;
- antiseborrheic or antiacne agents such as S-carboxymethylcysteine, S-benzylcysteamine, their salts or derivatives, or benzoyl peroxide.

Clearly, the skilled person will take care to select any compounds to be added to said compositions so that the advantageous properties intrinsically associated with the present invention are not or are not substantially altered by the envisaged addition.

Some examples will now be given by way of entirely non-limiting illustration of the production of compounds with general formula (I) of the invention, the biological activity results for these compounds and various formulations based on such compounds.

### EXAMPLE 1: 4-(1-Phenylmethanesulphonylpiperidin-4-yl)-benzene-1,3-diol

### a) 2,4-Bisbenzyloxy-1-bromobenzene

107 g (0.771 mol, 3 eq) of potassium carbonate (325 mesh) was added to a solution of 50.1 g (0.257 mol, 1 eq) of 97% 4-bromoresorcinol in 500 mL of acetone. The reaction medium was cooled to 5-10°C and 75 mL (0.630 mol, 2.45 eq) of benzyl bromide was added dropwise. The reaction medium was stirred at ambient temperature overnight then heated to 50 °C for 2 hours. The solvent was evaporated off then the residue was taken up in a water-ethyl acetate mixture. The aqueous phase was extracted with ethyl acetate, the organic phases were combined, washed with a saturated sodium chloride solution, dried over magnesium sulphate, filtered and evaporated. The residue (114 g) was chromatographed on silica gel (600 g), eluting with 90/10 heptane/dichloromethane.

94.4 g of 2,4-bisbenzyloxy-1-bromobenzene was obtained in the form of white crystals. Yield = 99%.

### b) Tert-butyl 4-(2,4-bisbenzyloxyphenyl)-4-hydroxy-piperidine-1-carboxylate

2 mL (5 mmol, 1.2 eq) of 2.5 M n-butyl lithium in hexane was added to a solution of 1.55 g (4 mmol, 1 eq) of 2,4-bisbenzyloxy-1-bromobenzene in 15 mL of methyltetrahydrofuran cooled to -70°C. The reaction medium was stirred at -70°C for 25 minutes and 1.0 g (5 mmol, 1.2 eq) of 1-boc-4-piperidone in solution in 10 mL of methyltetrahydrofuran was added. The reaction medium was stirred at -70°C for 1 hour then left overnight to come up to ambient temperature. 15 mL of a saturated solution of ammonium chloride supplemented with 2 mL of 2N hydrochloric acid was added to the reaction medium which was stirred vigorously for 20 minutes then was extracted with ethyl acetate. The organic phases were combined, dried over magnesium sulphate and evaporated. The residue was chromatographed on silica gel (AnaLogix SF40-80g column), eluting with 80/20 heptane/ethyl acetate.

730 mg of tert-butyl 4-(2,4-bisbenzyloxyphenyl)-4-hydroxypiperidine-1-carboxylate was obtained in the form of an oil. Yield = 37%.

### c) 4-(2,4-Dihydroxyphenyl)piperidine-1-carboxylic acid, tert-butyl ester

A mixture of 82.3 g (0.168 mmol, 1 eq) of tert-butyl 4-(2,4-bisbenzyloxyphenyl)-4-hydroxypiperidine-1-carboxylate in 620 mL of ethyl acetate and 210 mL of methanol in the presence of 16.4 g of 10% palladium on charcoal was stirred at 55°C under hydrogen at atmospheric pressure for 6 days. The reaction medium was filtered and the filtrate was concentrated and filtered. 38.9 g of tert-butyl 4-(2,4-dihydroxyphenyl)-piperidine-1-carboxylate was obtained in the form of an off-white solid. Yield = 79%.

### d) 4-(2,4-Dihydroxyphenyl)piperidinium hydrochloride

70 g of tert-butyl 4-(2,4-dihydroxyphenyl)piperidine-1-carboxylate was dissolved in 500 mL of ethyl acetate, then 150 mL of a solution of 4M hydrogen chloride in ethyl acetate was added. The reaction mixture was stirred for 2 hours at ambient temperature, then the solid formed was filtered. 36 g of 4-(2,4-dihydroxyphenyl)piperidinium hydrochloride was obtained. Yield = 69%.

### e) 4-(1-Phenylmethanesulphonylpiperidin-4-yl)benzene-1,3-diol

913 mg (4.79 mmol, 1.1 eq) of alpha-toluenesulphonyl chloride in solution in 5 mL of N,N-dimethylformamide was added dropwise to a solution of 1.0 g (4.35 mmol, 1 eq) of 4-(2,4-dihydroxyphenyl)-piperidinium hydrochloride in 15 mL of N,N-dimethylformamide in the presence of 758 µL (4.35 mmol, 1 eq) of diisopropylethylamine. The reaction medium was stirred at ambient temperature for 4 hours. The reaction medium was treated with water and extracted with ethyl acetate. The organic phases were combined, washed with water, dried over magnesium sulphate and evaporated off. The residue was chromatographed on silica gel eluted with heptane/ethyl acetate (gradient).

409 mg of 4-(1-phenylmethanesulphonylpiperidin-4-yl)-benzene-1,3-diol was obtained in the form of a white solid. MP = 207-208°C. Yield = 27%.

**¹****H NMR (DMSO, 400 MHz) :** 1.17 (m, 2H); 1.47 (d, *J* = 12 Hz, 2H); 2.73 (m, 3H); 3.63 (d, *J* = 12 Hz, 2H); 4.40 (s, 2H); 6.14 (dd, *J* = 8.4 & 2.4 Hz, 1H) ; 6.26 (d, *J* = 2.4 Hz, 1H) ; 6.79 (d, *J* = 7.9 Hz, 1H) ; 7.39 (m, 5H) ; 8.98 (s, 1H) ; 9.17 (s, 1H).

**¹³C NMR (DMSO, 100 MHz) :** 31.4, 33.9, 46.3, 54.3, 102.3, 106.0, 121.7, 126.7, 128.0, 128.3, 129.6, 130.9, 155.1, 156.2.

### EXAMPLE 2: 4-[1-(Toluene-4-sulphonyl)piperidin-4-yl]-benzene-1,3-diol

In a manner analogous to that of Example 1, but using p-toluenesulphonyl chloride in the place of alpha-toluenesulphonyl chloride, 4-[1-(toluene-4-sulphonyl)piperidin-4-yl]benzene-1,3-diol was obtained. MP = 206-207°C.

**¹****H NMR (DMSO, 400 MHz):** 1.60 (m, 2H); 1.70 (m, 2H); 2.19 (t, J = 10.9 Hz, 2H); 2.41 (s, 3H); 2.49 (m, 1H); 3.72 (d, *J* = 11.6 Hz, 1H); 6.12 (dd, *J* = 8.4 & 2.4 Hz, 1H); 6.22 (d, *J* = 2.4 Hz, 1H); 6.77 (d, *J* = 7.9 Hz, 1H); 7.46 (d, *J* = 8.4 Hz, 1H); 7.63 (d, *J* = 8.4 Hz, 1H), 8.98 (s, 1H); 9.10 (s, 1H).

**¹³****C NMR (DMSO, 100 MHz):** 20.99, 30.7, 33.7, 46.8, 102.3, 106.0, 121.5, 126.7, 127.5, 129.7, 132.4, 143.4, 155.2, 156.2.

### EXAMPLE 3: 4-[1-(Butane-1-sulphonyl)piperidin-4-yl]-benzene-1,3-diol

In a manner analogous to that of Example 1, but using butylsulphonyl chloride, 4-[1-(butane-1-sulphonyl)-piperidin-4-yl]benzene-1,3-diol was obtained. MP = 182-183°C. Yield = 33%.

**¹****H NMR (DMSO, 400 MHz) :** 0.90 (t, *J* = 7.2 Hz, 3H); 1.17 (m, 2H); 1.75-1.35 (m, 6H); 2.79 (m, 3H); 3.02 (t, *J* = 7.8 Hz, 2H); 3.66 (d, *J* = 11.6 Hz, 1H); 6.15 (dd, *J* = 8.4 & 2.0 Hz, 1H); 6.26 (d, *J* = 2.0 Hz, 1H); 6.82 (d, *J* = 7.9 Hz, 1H); 8.98 (s, 1H); 9.18 (s, 1H).

**¹³C NMR (DMSO, 100 MHz) :** 13.5, 21.0, 24.7, 31.4, 33.9, 46.1, 47.3, 102.4, 106.0, 121.8, 126.7, 155.2, 156.2.

### Example 4: Tyrosinase activity inhibition test

The activity of the inhibitors was measured using a lysate of B16F1 cells (murine melanoma line). In the presence of L-tyrosine substrate, the tyrosinase present in these cells catalyses the hydroxylation of L-tyrosine to L-DOPA then oxidation of the L-DOPA to dopaquinone. In the presence of MBTH (3-methyl-2-benzothiazolinone hydrazone), dopaquinone is trapped to form a pink complex which absorbs at 520 nm.

The B16F1 cells were cultivated in DMEM medium + 10% foetal calf serum + 10⁻⁹ M of αMSH for 4 days at 37°C under 7% CO₂. They were treated with trypsin, washed with PBS, counted and centrifuged. The pellet was taken up in an amount of 10⁷ cells/mL in lysis buffer (sodium phosphate, 10 mM pH 6.8 - Igepal 1%) and the suspension was treated with ultrasound for 10 seconds. After centrifuging for 30 minutes at 4000 rpm, the supernatant obtained constituted the cell lysate used as the source of tyrosinase in the enzymatic test.

The tests were carried out in duplicate in 384-well plates with a total volume of 50 µl. Each well contained:
- 40 µl of solution containing 1.25 mM of L-tyrosine, 6.25 µM of L-DOPA (cofactor) and 3.75 mM of MBTH in buffer B (sodium- phosphate 62.25 mM, pH 6.8 - 2.5% dimethylformamide);
- 5 µl of inhibitor diluted in DMSO;
- 5 µl of cell lysate diluted to half strength in Tris HCl buffer, 50 mM, pH 7.5.

The plate was incubated at 37°C and a spectrographic recording was produced at 520 nm after 6 hours incubation. In order to compensate for any absorption by the products, a corrected absorbance was used (absorbance at time 6 h - absorbance at time zero).

The inhibitors were dose-response tested in order to calculate the IC50 (dose inhibiting 50% of enzymatic activity).

A number of internal controls were added in each experiment:
- 100% activity control: the 5 µl of inhibitor was replaced with 5 µl of DMSO;
- 50% activity control: the 5 µl of inhibitor was replaced with 5 µl of phenylthiourea, 300 µM in DMSO;
- 0% activity control: the L-tyrosine substrate was replaced with buffer B.

The results obtained for the compounds of the invention are shown in Table A:

**Table A**

| **Name** | **Structure** | **Tyrosine hydroxylase/ Dopa oxidase IC50 (µM)** |
|---|---|---|
| 4-butylresorcinol (Rucinol) | 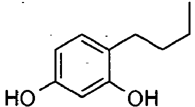 | 3 |
| Compound 2 | 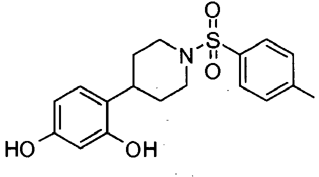 | 1.5 |

### Example 5: Melanogenesis inhibition test

The inhibition of melanogenesis was measured in MNT1 human melanoma cells using a protocol adapted from Reigner et al, Cell Mol Biol (1999) 45: 969-980. The test was based on the concomitant incorporation of 2 radiolabelled tracers: ¹⁴C-thiouracil is incorporated into neosynthesized melanin and reflects melanogenesis, while ³H-leucine is incorporated into proteins and reflects cell viability and, as a result, the toxicity of the test compounds.

The MNT1 cells were plated into 96-well plates in the presence of the test compounds and radioisotopes. After 24h incubation at 37°C, the cells were washed and the quantity of the 2 radioisotopes was measured. The test compounds were dose-response tested in order to calculate the IC50 inhibition of melanogenesis on the basis of the ¹⁴C incorporation which was normalized by the ³H incorporation. A cell toxicity IC50 was also calculated on the basis of the ³H incorporation.

This test could thus be used to differentiate products which specifically inhibit melanogenesis from those which are cytotoxic as regards melanocytes.

| **Name** | **Formula** | **IC50 melanogenesis** | **IC50 toxicity** |
|---|---|---|---|
| **4-butyl-resorcinol (Rucinol)** | 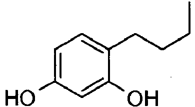 | 15 µM | 55 µM |
| Compound 1 | 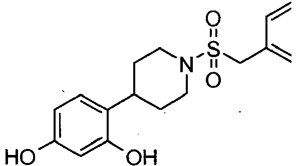 | 0.2 µM | >999 µM |

### Example 6: Formulations

This example illustrates various formulations based on the compounds of the invention.

### TOPICAL APPLICATION

| (a) Ointment | | |
|---|---|---|
| | • compound 2 | 0.020 g |
| | • isopropyl myristate | 81.700 g |
| | • fluid vaseline oil | 9.100 g |
| | • silica ("Aerosil 200") | 9.180 g |
| | | |

| (b) Ointment | | |
|---|---|---|
| | • compound 2 | 0.300 g |
| | • white vaseline codex | qsp 100 g |
| | | |

| (c) Non-ionic water-in-oil cream | | |
|---|---|---|
| | • compound 2 | 0.100 g |
| | • mixture of emulsified lanolin alcohols, | |
| | waxes and oils ("anhydrous eucerin") | 39.900 g |
| | • methyl para-hydroxybenzoate | 0.075 g |
| | • propyl para-hydroxybenzoate | 0.075 g |
| | • sterile demineralized water | qsp 100 g |
| | | |

| (d) Lotion | | |
|---|---|---|
| | • compound 2 | 0.100 g |
| | • polyethylene glycol (PEG 400) | 69.900 g |
| | • 95% ethanol | 30.000 g |
| | | |

| (e) Hydrophobic ointment | | |
|---|---|---|
| | • compound 2 | 0.300 g |
| | • isopropyl myristate | 36.400 g |
| | • silicone oil ("Rhodorsil 47V300") | 36.400 g |
| | • beeswax | 13.600 g |
| | • silicone oil ("Abil 300.000 cst") | qsp 100 g |
| | | |

| (f) Non-ionic water-in-oil cream | | |
|---|---|---|
| | • compound 2 | 1.000 g |
| | • cetyl alcohol | 4.000 g |
| | • glycerol monostearate | 2.500 g |
| | • PEG 50 stearate | 2.500 g |
| | • shea butter | 9.200 g |
| | • propylene glycol | 2.000 g |
| | • methyl para-hydroxybenzoate | 0.075 g |
| | • propyl para-hydroxybenzoate | 0.075 g |
| | • sterile demineralized water | qsp 100 g |

## Claims

1. Compounds with the following general formula (I): 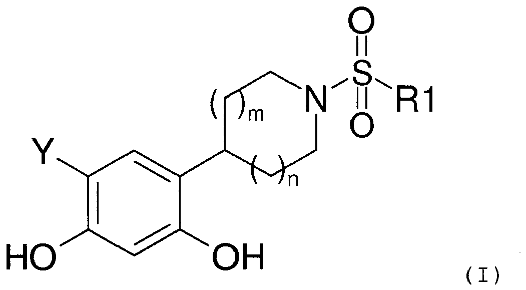 wherein
R1 represents:
• a C1-C8 alkyl radical;
• a C3-C8 cycloalkyl radical;
• a C4-C10 methylcycloalkyl radical;
• an aryl radical;
• a substituted aryl radical;
• a heteroaryl radical;
• a substituted heteroaryl radical;
• an aralkyl radical; or
• a C1-C5 alkoxy radical;
Y represents hydrogen or fluorine; and
m and n may independently take the values 0, 1 or 2;
as well as salts of the compounds with formula (I), and their enantiomeric forms.

2. Compound according to Claim 1, **characterized in that** it is in the form of a salt formed with a base selected from organic bases and inorganic bases.

3. Compound according to Claim 1 or 2, **characterized in that** it is in the form of a hydrate or a solvate.

4. Compound according to one of Claims 1 to 3, **characterized in that**:
• R1 represents:
• a substituted aryl radical;
• an aralkyl radical;
• a C3-C8 cycloalkyl radical; or
• a C4-C10 methylcycloalkyl radical;
• Y represents hydrogen or fluorine;
• m = 1 and n = 1;
as well as salts of said compounds with general formula (I) and their isomeric and enantiomeric forms.

5. Compound according to one of Claims 1 to 4, **characterized in that** it is selected from the group constituted by:
1: 4-(1-phenylmethanesulphonylpiperidin-4-yl)-benzene-1,3-diol;
2: 4-[1-(toluene-4-sulphonyl)piperidin-4-yl]-benzene-1,3-diol;
3: 4-[1-(butane-1-sulphonyl)piperidin-4-yl]-benzene-1,3-diol;
4: 4-(1-cyclohexylmethanesulphonylpiperidin-4-yl)-benzene-1,3-diol;
5: 4-[1-(2-phenylethanesulphonyl)piperidin-4-yl]-benzene-1,3-diol;
6: 1-[4-(2,4-dihydroxyphenyl)piperidine-1-sulphonylmethyl]-7,7-dimethyl-bicyclo[2.2.1]heptan-2-one;
7: 4-fluoro-6-[1-(toluene-4-sulphonyl)piperidin-4-yl]benzene-1,3-diol;
8: 4-fluoro-6-(1-phenylmethanesulphonylpiperidin-4-yl)benzene-1,3-diol;
9: 4-fluoro-6-[1-(2-phenylethanesulphonyl)-piperidin-4-yl]benzene-1,3-diol;
10:4-(1-cyclohexylmethanesulphonylpiperidin-4-yl)-6-fluorobenzene-1,3-diol;
11:4-[1-(butane-1-sulphonyl)piperidin-4-yl]-6-fluorobenzene-1,3-diol;
12:4-[1-(toluene-4-sulphonyl)pyrrolidin-3-yl]-benzene-1,3-diol;
13:4-(1-phenylmethanesulphonylpyrrolidin-3-yl)-benzene-1,3-diol;
14:4-[1-(2-phenylethanesulphonyl)pyrrolidin-3-yl]benzene-1,3-diol;
15:4-fluoro-6-[1-(2-phenylethanesulphonyl)-pyrrolidin-3-yl]benzene-l,3-diol;
16:4-(1-cyclohexylmethanesulphonylpyrrolidin-3-yl)benzene-1,3-diol;
17:4-[1-(butane-1-sulphonyl)pyrrolidin-3-yl]-benzene-1,3-diol.

6. Compound according to any one of Claims 1 to 5, as a drug.

7. Compound according to Claim 6, **characterized in that** said compound has a tyrosinase inhibiting activity.

8. Compound as a drug according to Claim 6 or 7, for use in the treatment and/or prevention of pigmentary disorders.

9. Compound for use according to Claim 8, **characterized in that** the pigmentary disorders are selected from melasma, chloasma, lentigines, senile lentigo, irregular hyperpigmentations linked to photo-ageing, freckles, post-inflammatory hyperpigmentations due to abrasion, a burn, a scar, dermatosis, or a contact allergy; naevi, hyperpigmentations with genetic determinism, hyperpigmentations of metabolic or drug origin, melanomas or any other hyperpigmentary lesion.

## Patentansprüche

1. Verbindungen mit der folgenden allgemeinen Formel (I) : 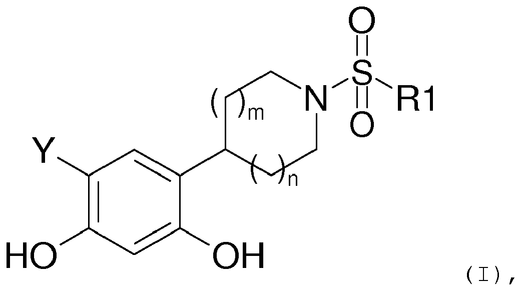 wobei
R1 darstellt:
• ein C1-C8 Alkylradikal;
• ein C3-C8 Cycloalkylradikal;
• ein C4-C10 Methylcycloalkylradikal;
• ein Arylradikal;
• ein substitutiertes Arylradikal;
• ein Heteroarylradikal;
• ein substitutiertes Heteroarylradikal;
• ein Aralkylradikal; oder
• ein C1-C5 Alkoxyradikal;
Y Wasserstoff oder Fluor darstellt; und
m und n können unabhängig voneinander die Werte 0, 1 oder 2 annehmen;
sowie Salze der Verbindungen mit der Formel (I), und ihre enantiomere Formen.

2. Verbindung nach Anspruch 1, da**dadurch gekennzeichnet, dass** es in der Form eines Salzes gebildet ist mit einer Base ausgewählt aus organischen Basen und anorganischen Basen.

3. Verbindung nach Anspruch 1 oder 2, **dadurch gekennzeichnet, dass** die Verbindung in der Form eines Hydrats oder eines Solvats ist.

4. Verbindung nach einem der Ansprüche 1 bis 3, **dadurch gekennzeichnet, dass**:
• R1 darstellt:
• ein substitutiertes Arylradikal;
• ein Aralkylradikal;
• ein C3-C8 Cycloalkylradikal; oder
• ein C4-C10 Methylcycloalkylradikal;
• Y Wasserstoff oder Fluor darstellt;
• m = 1 und n = 1;
sowie Salze und die Verbindungen mit allgemeiner Formel (I) und ihre isomeren und enantiomeren Formen.

5. Verbindung nach einem der Ansprüche 1 bis 4, **dadurch gekennzeichnet, dass** die Verbindung ausgewählt ist aus der Gruppe bestehend aus:
1: 4-(1-Phenylmethansulfonylpiperidin-4-yl)-Benzen-1,3-diol;
2: 4-[1-(Toluen-4-sulfonyl)piperidin-4-yl]-Benzen-1,3-diol;
3: 4-[1-(Butan-1-sulfonyl)piperidin-4-yl]-Benzen-1,3-diol;
4: 4-(1-Cyclohexylmethansulfonylpiperidin-4-yl)-Benzen-1,3-diol;
5: 4-[1-(2-Phenylethansulfonyl)piperidin-4-yl]-Benzen-1,3-diol;
6: 1-[4-(2,4-Dihydroxyphenyl)piperidin-1-sulfonylmethyl]-7,7-dimethyl-bicyclo[2.2.1]Heptan-2-on;
7: 4-Fluoro-6-[1-(toluen-4-sulfonyl)piperidin-4-yl]Benzen-1,3-diol;
8: 4-Fluoro-6-(1-phenylmethansulfonylpiperidin-4-yl)Benzen-1,3-diol;
9: 4-Fluoro-6-[1-(2-phenylethansulfonyl)-piperidin-4-yl]Benzen-1,3-diol;
10:4-(1-Cyclohexylmethansulfonylpiperidin-4-yl)-6-Fluorobenzen-1,3-diol;
11:4-[1-(Butan-1-sulfonyl)piperidin-4-yl]-6-Fluorobenzen-1,3-diol;
12:4-[1-(Toluen-4-sulfonyl)pyrrolidin-3-yl]-Benzen-1,3-diol;
13:4-(1-Phenylmethansulfonylpyrrolidin-3-yl)-Benzen-1,3-diol;
14:4-[1-(2-Phenylethansulfonyl)pyrrolidin-3-yl]Benzen-1,3-diol;
15:4-Fluoro-6-[1-(2-phenylethansulfonyl)-pyrrolidin-3-yl]Benzen-1,3-diol;
16:4-(1-Cyclohexylmethansulfonylpyrrolidin-3-yl)Benzen-1,3-diol;
17:4-[1-(Butan-1-sulfonyl)pyrrolidin-3-yl]-Benzen-1,3-diol.

6. Verbindung nach einem der Ansprüche 1 bis 5 als ein Arzneimittel.

7. Verbindung nach Anspruch 6, **dadurch gekennzeichnet, dass** die Verbindung eine Tyrosinase inhibierende Aktivität hat.

8. Verbindung als ein Arzneimittel nach Anspruch 6 oder 7, zur Verwendung für die Behandlung und/oder Prävention von Pigmentstörungen.

9. Verbindung zur Verwendung nach Anspruch 8, **dadurch gekennzeichnet, dass** die Pigmentstörungen ausgewählt sind aus Melasma, Chloasma, Leberflecke, Lentigo senilis, irreguläre Hyperpigmentierung verbunden mit lichtbedingter Alterung, Sommersprossen, post-inflammatorische Hyperpigmentierung durch Abrasion, eine Verbrennung, eine Narbe, Dermatosis oder eine Kontkatallergie; Naevi, Hyperpigmentierungen mit genetischem Determinismus, Hyperpigmentierungen des Stoffwechsels oder Arzneimittelursprungs, Melanome oder eine andere hyperpigmentäre Verletzung.

## Revendications

1. Composés répondant à la formule générale (I) suivante : 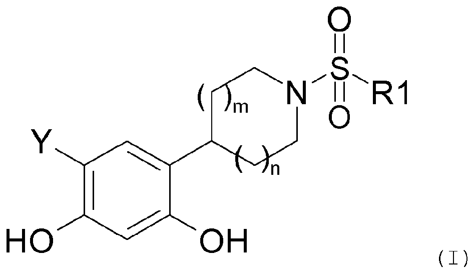 dans laquelle
R1 représente :
• un radical alkyle en C₁ à C₈;
• un radical cycloalkyle en C₃ à C₈;
• un radical méthylcycloalkyle en C₄ à C₁₀ ;
• un radical aryle ;
• un radical aryle substitué ;
• un radical hétéroaryle ;
• un radical hétéroaryle substitué ;
• un radical aralkyle ; ou
• un radical alcoxy en C₁ à C₅ ;
Y représente un atome d'hydrogène ou un atome de fluor ; et
m et n peuvent indépendamment prendre la valeur 0, 1 ou 2 ;
ainsi que les sels des composés répondant à la formule (I), et leurs formes énantiomères.

2. Composé selon la revendication 1, **caractérisé en ce qu'**il est sous la forme d'un sel formé avec une base choisie parmi les bases organiques et les bases inorganiques.

3. Composé selon la revendication 1 ou 2, **caractérisé en ce qu'**il est sous la forme d'un hydrate ou d'un solvate.

4. Composé selon l'une quelconque des revendications 1 à 3, **caractérisé en ce que** :
R1 représente :
• un radical aryle substitué ;
• un radical aralkyle ;
• un radical cycloalkyle en C₃ à C₈; ou
• un radical méthylcycloalkyle en C₄ à C₁₀ ;
Y représente un atome d'hydrogène ou de fluor :
m = 1 et n = 1 ;
ainsi que les sels desdits composés répondant à la formule générale (I) et leurs formes isomères et énantiomères.

5. Composé selon l'une quelconque des revendications 1 à 4, **caractérisé en ce qu'**il est choisi dans le groupe constitué par :
1 : 4-(1-phénylméthanesulfonylpipéridin-4-yl)-benzène-1,3-diol ;
2 : 4-[1-(toluène-4-sulfonyl)pipéridin-4-yl]-benzène-1,3-diol ;
3 : 4-[1-(butane-1-sulfonyl)pipéridin-4-yl]-benzène-1,3-diol ;
4 : 4-(1-cyclohexylméthanesulfonylpipéridin-4-yl)-benzène-1,3-diol ;
5 : 4-[1-(2-phényléthanesulfonyl)pipéridin-4-yl]-benzène-1,3-diol ;
6 : 1-[4-(2,4-dihydroxyphényl)pipéridine-1-sulfonylméthyl]-7,7-diméthyl-bicyclo[2.2.1]heptan-2-one ;
7 : 4-fluoro-6-[1-(toluène-4-sulfonyl)pipéridin-4-yl]benzène-1,3-diol ;
8 : 4-fluoro-6-(1-phénylméthanesulfonylpipéridin-4-yl)benzène-1,3-diol ;
9 : 4-fluoro-6-[1-(2-phényléthanesulfonyl)-pipéridin-4-yl]benzène-1,3-diol ;
10 : 4-(1-cyclohexylméthanesulfonylpipéridin-4-yl)-6-fluorobenzène-1,3-diol ;
11 : 4-[1-(butane-1-sulfonyl)pipéridin-4-yl]-6-fluorobenzène-1,3-diol ;
12 : 4-[1-(toluène-4-sulfonyl)pyrrolidin-3-yl]-benzène-1,3-diol ;
13 : 4-(1-phénylméthanesulfonylpyrrolidin-3-yl)-benzène-1,3-diol ;
14 : 4-[1-(2-phényléthanesulfonyl)pyrrolidin-3-yl]benzène-1,3-diol ;
15 : 4-fluoro-6-[1-(2-phényléthanesulfonyl)-pyrrolidin-3-yl]benzène-1,3-diol ;
16 : 4-(1-cyclohexylméthanesulfonylpyrrolidin-3-yl)benzène-1,3-diol ;
17 : 4-[1-(butane-1-sulfonyl)pyrrolidin-3-yl]-benzène-1,3-diol.

6. Composé selon l'une quelconque des revendications 1 à 5, en tant que médicament.

7. Composé selon la revendication 6, **caractérisé en ce que** ledit composé a une activité inhibitrice de la tyrosinase.

8. Composé en tant que médicament selon la revendication 6 ou 7, pour une utilisation dans le traitement et/ou la prévention de troubles de la pigmentation.

9. Composé pour une utilisation selon la revendication 8, **caractérisé en ce que** les troubles de la pigmentation sont choisis parmi le mélasma, le chloasma, les lentigines, le lentigo sénile, les hyperpigmentations irrégulières liées au photo-vieillissement, les taches de rousseur, les hyperpigmentations post-inflammatoires dues à une abrasion, une brûlure, une cicatrice, une dermatose ou une allergie de contact ; les nevi, les hyperpigmentations à déterminisme génétique, les hyperpigmentations d'origine métabolique ou médicamenteuse, les mélanomes ou toute autre lésion hyperpigmentaire.
